# EUROPEAN PATENT APPLICATION

(11) **EP 3 648 111 A1**
(43) Date of publication of application: **06.05.2020**
(21) Application number: 19203454.4
(22) Date of filing: 16.10.2019
(51) Int. Cl.: G16H 10/20

(54) **ELECTRONIC CLINICAL CASE REPORTING**

(30) Priority: 31.10.2018 FI 20185918
(71) Applicant: CRF Box Oy, 00100 Helsinki (FI)
(72) Inventor: Lehtomäki, Riku, 00100 Helsinki (FI); Haaman, Ari, 00100 Helsinki (FI); Huminiuc, Mihai, 00100 Helsinki (FI); Patras, Silviu, 00100 Helsinki (FI)
(74) Representative: Espatent Oy

(57) **Abstract**

A handheld device that has a user interface equipped at least with a display. The device provides a user with a home screen on a display of the handheld device by presenting one or more electronic case reporting questionnaires or links to one or more electronic case reporting questionnaires. The device starts an allowable interfacing application if not running already. The device provides the allowable interfacing application with such limited access to the user interface that a return key is reserved to be unusable by the allowable interfacing application and again provides the user with the home screen on the display of the handheld device in response to meeting one or more return conditions.

## Description

### TECHNICAL FIELD

The present invention generally relates to electronic clinical case reporting.

### BACKGROUND ART

This section illustrates useful background information without admission of any technique described herein representative of the state of the art.

Clinical trials require use of strictly validated questionnaires, instruments. The instruments applied to particular clinical trial are predetermined and designed for a particular phase of the clinical trial. In case of electronic reporting, presentation of the instruments and collecting of user input are implemented using an electronic tool, such as a handheld device that has suitable computing and user interfacing equipment.

Handheld computing devices are also suitable for interfacing with various test equipment that may be needed in conjunction with the clinical trial questionnaires. For example, blood sugar tester, pulse sensor, blood pressure sensor, or thermometer can be connected by wires or wirelessly with the handheld device for directly receiving measurements that may be useful for the clinical trial. All kinds of wearables can be used for collecting various types of data, wherein the wearable can be connected to the handheld device for synchronizing collected data to the handheld device, typically by means of a dedicated software installed on the handheld device. On the other hand, handheld devices could also be used as controllers for dispensers such as an insulin pump, medicine pump, or inhalator, and / or for medical devices such as heart pacer.

In a clinical trial setup, the handheld device is typically configured and delivered to the clinical trial site by a vendor. The handheld device configuration includes installing necessary collector application, such as an electronic diary, required instruments and configuring the device to be operated in kiosk mode. In kiosk mode access to file system of the handheld device is prevented. The handheld device can be provisioned as required, e.g., due to a trial protocol amendment, with required application updates and new questionnaires.

Modern mobile phones and tablet computers provide very ubiquitous and capable platform for running computer programs of all sorts, including clinical trials. However, such devices are typically open to modifications that may prejudice intended operation, e.g. validated operation of clinical trial applications. A device handed out for use as a clinical trial platform may even be reconfigured by an end-user to operate as a gaming device and some of the installed software either intentionally or inadvertently prevents or hampers with the clinical trial.

There are various versions of parental control applications and operating system features intended to enable parents control and monitor use of smart phones. For example, Google Family Link™ and Screen Time by Screen Time Labs enable parents to monitor use of a given mobile phone and to allow or prevent particular applications and content as they see fit to their children. These services run on a mobile device a background application that has sufficient rights to report use of and to stop other applications as defined by the parents. The parental control applications can be easily installed by a user, but also uninstalled as easily.

There are also some operating system features for compartmentalizing particular data or applications of a smart phone, such as Secure Folder by Samsung®. Sensitive applications and data are sandboxed so that proper a PIN code or fingerprint authenticates access. While the phone operates in the Secure Folder, applications and data placed therein are accessible to the user. Once the user returns to normal mode (e.g., by pressing home key or back button), the user has to re-authenticate herself.

Whereas the secure folder takes an authentication to gain elevated access, there are also some cases in which access rights are lowered and authentication is required to resume normal rights. Lumia® 820 and 920 had a Kids Corner in which a user (child) was given access to programs and data predetermined by the user of the phone.

Third-party software exists for various uses. For instance, external equipment may be interfaced using such software, which may also require user interfacing. It is possible to tailor such third-party software to co-operate with the clinical trial equipment. However, such changes may require further validation of the trial instruments or third-party software, raise liability questions and risk producing programming errors and therefore require increased testing.

It is an object of the present invention to facilitate electronic clinical case reporting, or to improve co-operating of software of different parties in clinical case reporting or at least to provide a new technical alternative for electronic clinical case reporting.

### SUMMARY

According to a first example aspect of the invention there is provided a method for controlling a handheld device that comprises a user interface equipped at least with a display, the method comprising:
causing the handheld device to provide a user with a home screen on a display of the handheld device;
causing the handheld device to start an allowable interfacing application if not running already;
causing the handheld device to provide the allowable interfacing application with such limited access to the user interface that a return key is reserved to be unusable by the allowable interfacing application; and
causing the handheld device to again provide the user with the home screen on the display of the handheld device in response to meeting one or more return conditions.

The method may further comprise causing the handheld device to identify an allowable interfacing application stored in the handheld device;

The method may further comprise causing the handheld device to receive an identification of the allowable interfacing application. The identification may comprise particulars, such as download link and/or credentials, for obtaining the allowable interfacing application. The allowable interface application may be stored in a mounting packet and installable by the handheld device.

The method may further comprise receiving the allowable application from an external entity. The external entity may be hosted by an organization in charge of the electronic clinical case reporting for which the handheld device is used. The external entity may be a discreet server. The external entity may be a distributed server. The external entity may comprise virtualized or cloud-based functions.

The providing of the user with the home screen may comprise presenting one or more electronic case reporting questionnaires or links to one or more electronic case reporting questionnaires.

According to a second example aspect of the invention there is provided a computer program comprising computer executable program code which, when executed, controls a handheld device that comprises a user interface equipped at least with a display, to perform a method of the first example aspect.

The providing of the user with the home screen may comprise presenting one or more electronic case reporting questionnaires or links to one or more electronic case reporting questionnaires.

The one or more return conditions may comprise detecting that the user has used the return key. The one or more return conditions may comprise that a timer has expired. The one or more return conditions may comprise that a remote command has been received by the handheld device. The one or more return conditions may comprise that the allowable interfacing application has ceased to use the display. The one or more conditions may comprise that the allowable interfacing application has stopped sending data, operating, has lost connection to the handheld device. The one or more conditions may comprise that an external device communicating with the allowable interfacing application has ceased communicating properly with the handheld device.

The return key may be a soft key. The soft key may comprise a legend shown by the display. The soft key may comprise a touch screen region or a hard key associated with the legend. Alternatively, the return key may be a hard key. The return key may be independent of any return function control of an operating system of the handheld device. A return key can be actuated by an audio command.

The handheld device may be a smart phone. Alternatively, the handheld device may be a tablet computer.

The handheld device may be provisioned with the allowable interfacing application over the air.

The allowable interfacing application may be configured to interface with the user.

The allowable interfacing application may be configured to interface with external equipment. The external equipment may be connected by wires and/or wirelessly with the handheld device.

The external equipment may comprise measuring equipment. The measuring equipment may comprise a blood sugar tester or a glucometer. The glucometer may be a continuous glucose monitoring (CGM) device. The measuring equipment may comprise a pulse sensor. The measuring equipment may comprise a blood pressure sensor. The measuring equipment may comprise a thermometer. The measuring equipment may comprise a device with an inertia sensor. The measuring equipment may comprise one or more ambulatory electrocardiographs. The measuring equipment may comprise an accelerometer. The measuring equipment may comprise a gyroscope. The measuring equipment may comprise an internal, e.g., ingestible, pH monitor. The measuring equipment may comprise an ambulatory blood pressure monitor. The measuring equipment may comprise a heart rate monitor. The measuring equipment may comprise a pressure sensor, e.g., a blood pressure sensor. The measuring equipment may comprise an instrumented walkway. The measuring equipment may comprise a medication adherence monitor. The measuring equipment may comprise a geolocation monitor, e.g., a satellite position sensor. The measuring equipment may comprise one or more wearable sensors. The measuring equipment may comprise one or more implantable sensors. The method may be further comprise causing the handheld device to receive and buffer information from the measurement equipment. The method may be further comprise causing the handheld device to transmit (e.g., wirelessly) information received from the measurement information to a back end. The back end may comprise one or more servers. The back end may be implemented by cloud computing. Alternatively, the allowable interfacing application may be configured to receive information from the measurement equipment. The allowable interfacing application may be configured to send (e.g., wirelessly) the received information to the back end.

The external equipment may comprise an actuation controller. The actuation controller may control producing of action that is capable of triggering a physical response in a person. The external equipment may comprise a medical dispenser. The external equipment may comprise a cardiac device. The external equipment may comprise a nerve signal stimulating device. The external equipment may comprise a cardioverter defibrillator.

The method may be further comprise causing the handheld device to close the allowable interfacing application.

The method may be further comprise causing the handheld device to restart the allowable interfacing application.

According to a third example aspect of the invention there is provided handheld device comprising a memory that comprises the computer program of the first example aspect and a processor configured to execute the computer program.

According to a fourth example aspect of the invention there is provided a handheld device comprising:
a user interface equipped at least with a display;
means for causing the handheld device to provide a user with a home screen on a display of the handheld device;
means for causing the handheld device to start an allowable interfacing application if not running already;
means for causing the handheld device to provide the allowable interfacing application with such limited access to the user interface that a return key is reserved to be unusable by the allowable interfacing application; and
means for causing the handheld device to again provide the user with the home screen on the display of the handheld device in response to meeting one or more return conditions.

The handheld device may further comprise means for causing the handheld device to identify an allowable interfacing application stored in the handheld device.

The providing of the user with the home screen may comprise presenting one or more electronic case reporting questionnaires or links to one or more electronic case reporting questionnaires.

According to a fifth example aspect of the invention there is provided a method for digital case reporting, comprising:
performing the method of the second example aspect; and
transferring data reported by the allowable interfacing application from a respective first back-end data storage to a second back-end data storage that differs from the first back-end storage.

The transferring of data between the first and second back-end storages may be implemented using a server integration.

The method of the fifth example aspect may comprise monitoring by the second back-end the operation of the handheld device. The method may further comprise commanding the handheld device to cause starting or stopping the allowable interfacing application.

According to a sixth example aspect of the invention there is provided a computer program comprising computer executable program code, which when executed by a computer, causes the computer to perform or cause the method of the fifth example aspect.

According to a seventh example aspect of the invention there is provided a computer program product comprising a non-transitory computer readable medium having the computer program of the first or sixth example aspect stored thereon. The memory medium may be a non-transitory computer readable medium.

Any foregoing memory medium may comprise a digital data storage such as a data disc or diskette, optical storage, magnetic storage, holographic storage, opto-magnetic storage, phase-change memory, resistive random access memory, magnetic random access memory, solid-electrolyte memory, ferroelectric random access memory, organic memory or polymer memory. The memory medium may be formed into a device without other substantial functions than storing memory or it may be formed as part of a device with other functions, including but not limited to a memory of a computer, a chip set, and a sub assembly of an electronic device.

Different non-binding example aspects and embodiments of the present invention have been illustrated in the foregoing. The embodiments in the foregoing are used merely to explain selected aspects or steps that may be utilized in implementations of the present invention. Some embodiments may be presented only with reference to certain example aspects of the invention. It should be appreciated that corresponding embodiments may apply to other example aspects as well.

### BRIEF DESCRIPTION OF THE DRAWINGS

Some example embodiments of the invention will be described with reference to the accompanying drawings, in which:
- Fig. 1: shows a schematic drawing of a system according to an embodiment of the invention;
- Fig. 2: shows a block diagram of a server according to an embodiment of the invention;
- Fig. 3: shows a block diagram of a terminal of Fig. 1;
- Fig. 4: shows a simplified block diagram of a method according to an embodiment of the invention;
- Fig. 5: shows a simplified view of a user terminal of an embodiment; and
- Fig. 6: shows a flow chart of an embodiment.

### DETAILED DESCRIPTION

In the following description, like reference signs denote like elements or steps.

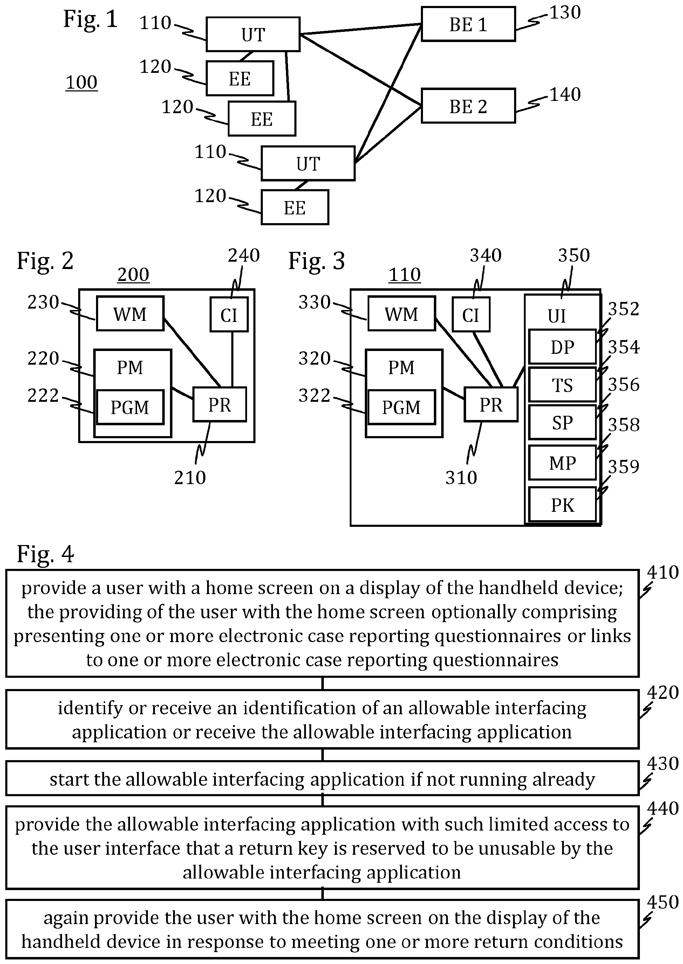

Fig. 1 shows a schematic drawing of a system 100 according to an embodiment of the invention. The system comprises a plurality of user terminals 110; optionally a plurality of external equipment 120 of various types and implementations located at different user terminals 110; a first back end 130; and a second back end 140. The first and second back ends 130, 140 may comprise respective data storages. The user terminals 110 have data connectivity, e.g. through the Internet, to the first and second back ends 130, 140. The back ends have data connectivity also between each other, either directly or via one or more proxies. Either or both of the back ends 130 first and second back ends 130, 140 can be implemented using one or more central or distributed servers, computer clouds, virtualization and/or cluster computing.

Fig. 2 shows a block diagram of a server 200 according to an embodiment of the invention, suited for implementing the first and/or second back end 130, 140. The server comprises one or more processors 210; a persistent data storage 220; a work memory 230; and a communication interface 240. The persistent data storage comprises computer program code 242, such as code for implementing an operating system and various applications.

The persistent data storage 220 comprises, e.g., one or more hard drives; optical storages; tape drives; solid state drives; USB memories; and/or any other elements. The persistent data storage 220 is in an embodiment configured to maintain data over periods longer than six months or for more than one hour without power supply.

The work memory may comprise one or more random access memories, such as one or more random access memory circuitries.

Fig. 3 shows a block diagram of an embodiment of the terminal 110. The terminal 110 comprises a processor 310; a persistent data storage 320 comprising computer program code 322; a work memory 330; a communication interface 340; and a user interface 350. The processor may be implemented using similar or same parts as explained for the processor 210 of the server. Also the persistent memory 320 and work memory 330 may be implemented using same or similar parts as explained for corresponding elements of the server 200.

The user interface 350 comprises, for example, any one or more of: a display 352; a touch screen 354, e.g., on the display 352 and/or around the display 352; a speaker 356; a microphone 358; and one or more physical keys 359 that may be implemented, e.g., as push buttons; rotatable keys; sliding keys; rocker keys; touch keys.

The terminal 110 may be capable of speech recognition, e.g., using the processor 310 and suitable speech recognition software stored in the persistent data storage 320.

The computer program code 322 defines an operating system. Based on the operating system, the terminal 110 is capable of running one or more programs. In an embodiment, the terminal is configured to first launch a control program referred to herein as a KIOSK program through which one or more other programs can be launched and their rights can be controlled, e.g. access rights to the user interface. The one or more other programs may be launched while an allowable interfacing application is running.

In an example embodiment, the control program allows a third party program to be launched and to access the user interface except for a reserved portion of the user interface, which is kept for the purpose of the control program. Such a reserved portion may comprise, for example, a portion of the display 352 (either entirely or overlay something by the control program onto underlying third party content) and a user input control (e.g., touch screen portion matching with the portion of the display of the reserved portion; an adjacent hard key; and/or a speech recognition command).

Fig. 4 shows a simplified block diagram of a method according to an embodiment of the invention. The method causes a handheld device that comprises a user interface equipped at least with a display, to perform one or more of:
410. provide a user with a home screen on a display of the handheld device; the providing of the user with the home screen comprising in an embodiment presenting one or more electronic case reporting questionnaires or links to one or more electronic case reporting questionnaires;
420. identify or receive an identification of an allowable interfacing application or receive the allowable interfacing application;
430. start the allowable interfacing application if not running already;
440. provide the allowable interfacing application with such limited access to the user interface that a return key is reserved to be unusable by the allowable interfacing application; and
450. again provide the user with the home screen on the display of the handheld device in response to meeting one or more return conditions.

In an embodiment, the method of Fig. 4 is caused to be performed by the control program.

The identifying of an allowable interfacing application may be based on receiving the identification of the allowable interfacing application, for example, from an external entity. In an embodiment, the identification comprises particulars, such as download link and/or credentials, for obtaining the allowable interfacing application. In an embodiment, the allowable interface application is stored in a mounting packet and installable by the handheld device.

In an example embodiment, the method further comprises receiving the allowable application from an external entity.

In an embodiment, the external entity is hosted by an organization in charge of the electronic clinical case reporting for which the handheld device is used. In an embodiment, the external entity is a discreet server. In an embodiment, the external entity is a distributed server. In an embodiment, the external entity comprises virtualized or cloud-based functions.

In an embodiment, the one or more return conditions comprise any one or more of: detecting that the user has used the return key; a timer has expired; a remote command has been received by the handheld device; the allowable interfacing application has ceased to use the display; the allowable interfacing application has stopped sending data, operating, has lost connection to the handheld device; an external device communicating with the allowable interfacing application has ceased communicating properly with the handheld device.

In an embodiment, the return key comprises any one or more of: a soft key; a legend shown by the display; a touch screen region or a hard key associated with the legend. In an embodiment, the return key is a hard key. In an embodiment, the return key is independent of any return function control of an operating system of the handheld device. The return key can be actuated by an audio command.

In an embodiment, the handheld device is a smart phone or a tablet computer.

In an embodiment, the handheld device is provisioned with the allowable interfacing application over the air.

The allowable interfacing application may be configured to interface with the user.

In an embodiment, the allowable interfacing application is configured to interface with external equipment. In an embodiment, the external equipment is connected by wires and/or wirelessly with the handheld device.

In an embodiment, the external equipment 120 comprises measuring equipment, including any one or more of: a blood sugar tester or a glucometer that may be a continuous glucose monitoring (CGM) device; a pulse sensor; a blood pressure sensor; a thermometer; an inertia sensor; one or more ambulatory electrocardiographs; an accelerometer a gyroscope an internal, e.g., ingestible, pH monitor; an ambulatory blood pressure monitor; heart rate monitor; an instrumented walkway; a medication adherence monitor; a geolocation monitor, e.g., a satellite position sensor. In an embodiment, the measuring equipment comprises one or more wearable sensors. In an embodiment, the measuring equipment comprises one or more implantable sensors. In an embodiment, the measuring equipment is connectable to the user terminal 110 wirelessly, e.g., using Bluetooth or wireless LAN (e.g., any IEEE 802.11b compatible version) and/or by wired connection, such as universal serial bus (USB), RS-232, and/or IEEE-1394. In an embodiment, the measuring equipment is autonomously operable, whereas in another embodiment, the measuring equipment requires access to one or more components of the user terminal 110 to operate. For example, the measuring equipment may employ the processor 310 and/or memory 330 of the user terminal 110.

In an embodiment, the handheld device receives and buffers information from the measurement equipment. In an embodiment, the handheld device transmits (e.g., wirelessly) information received from the measurement information to the second back end 140 or to the first back end 130. Any one or more of the first and second back end may comprise one or more servers or be implemented by cloud computing. Alternatively, the allowable interfacing application may be configured to receive information from the measurement equipment. In an embodiment, the allowable interfacing application is configured to cause sending (e.g., wirelessly) the received information to the second back end 140.

In an embodiment, the external equipment 120 comprises an actuation controller. The actuation controller may control producing of action that is capable of triggering a physical response in a person. The external equipment may comprise a medical dispenser. In an embodiment, the external equipment comprises any one or more of: a cardiac device; a nerve signal stimulating device; and/or a cardioverter defibrillator.

In an embodiment, the handheld device closes the allowable interfacing application and/or to restarts the allowable interfacing application.

In an embodiment, the transferring of data between the first and second back-end storages is implemented using a server integration.

In an embodiment, the operation of the handheld device is monitored by the second back-end, e.g. by commanding the handheld device to cause starting or stopping the allowable interfacing application.

A skilled person appreciates that in addition to the elements shown in Figure 2 or 3, the server 200 and/or the user terminal 110 may comprise other elements, such as microphones, displays, as well as additional circuitry such as input/output (I/O) circuitry, memory chips, application-specific integrated circuits (ASIC), processing circuitry for specific purposes such as source coding/decoding circuitry, channel coding/decoding circuitry, ciphering/deciphering circuitry, and the like.

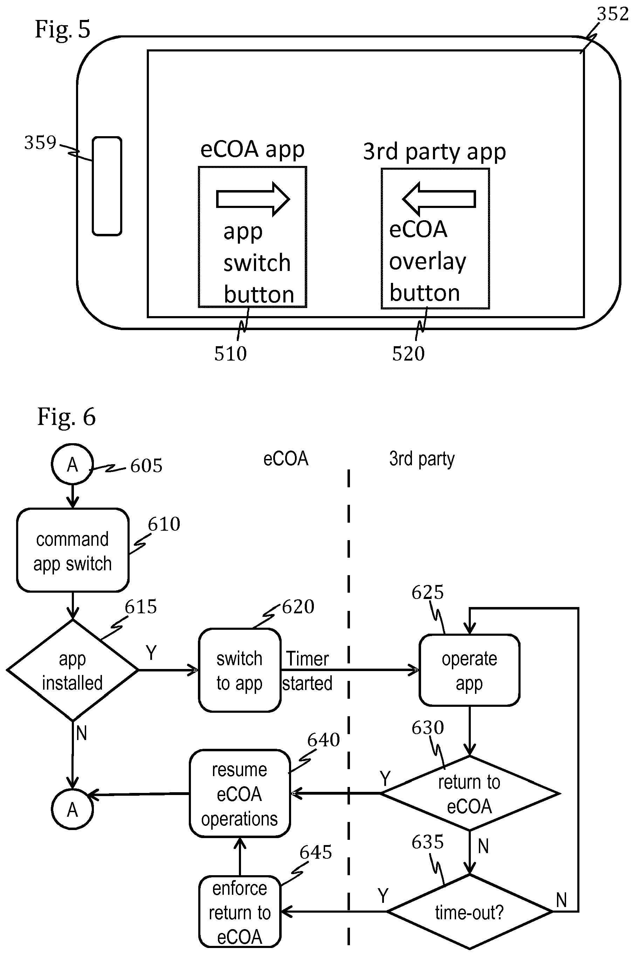

Fig. 5 shows a simplified view of a user terminal of an embodiment. The display 352 presents an app switch button 510 provided by the control program for switching to a third party application (the allowable interfacing application) and another button 520 for resuming the display to the control program. Fig. 5 further presents a physical key 359 that in one embodiment is entirely disabled.

Fig. 6 shows a flow chart of an embodiment. The flow chart begins from step A 605 in which the control program is running. A command for app switch is detected 610 (e.g., by user using the app switch button 510) or, e.g., from a remote command. The application is switched 620 (e.g., allowable interfacing application is given rights to use most of the display 352). In an embodiment, a timer is also started on switching the application. The switched application is then operated 625 until a command is detected for returning to the control program in 630 or the timer runs out at timeout check 635. If a command for returning to the control program is detected in 630, then the process continues to step 640 in which the control program operations are resumed and then the process resumes to step A 605. On the other hand, if the operation of the switched application ends to timeout of the timer, a timeout is detected 635 and the process continues to step 645 in which return to the control program is enforced and then the process continues to steps 640 and back to A 605.

Any processor referred to by this document may be, e.g., a central processing unit (CPU), a microprocessor, a digital signal processor (DSP), a graphics processing unit, an application specific integrated circuit (ASIC), a field programmable gate array, a microcontroller or a combination of such elements. Any processor may employ plural discrete parts, comprising any one or more of those listed in the previous sentence.

Various embodiments have been presented. It should be appreciated that in this document, words comprise, include and contain are each used as open-ended expressions with no intended exclusivity.

The foregoing description has provided by way of non-limiting examples of particular implementations and embodiments of the invention a full and informative description of the best mode presently contemplated by the inventors for carrying out the invention. It is however clear to a person skilled in the art that the invention is not restricted to details of the embodiments presented in the foregoing, but that it can be implemented in other embodiments using equivalent means or in different combinations of embodiments without deviating from the characteristics of the invention. The abstract is incorporated by reference as an example embodiment.

Furthermore, some of the features of the afore-disclosed embodiments of this invention may be used to advantage without the corresponding use of other features. As such, the foregoing description shall be considered as merely illustrative of the principles of the present invention, and not in limitation thereof. Hence, the scope of the invention is only restricted by the appended patent claims.

## Claims

1. A method for controlling a handheld device that comprises a user interface equipped at least with a display, the method comprising:
causing the handheld device to provide a user with a home screen on a display of the handheld device; and performing a process comprising:
causing the handheld device to start an allowable interfacing application if not running already;
causing the handheld device to provide the allowable interfacing application with such limited access to the user interface that a return key is reserved to be unusable by the allowable interfacing application; and
causing the handheld device to again provide the user with the home screen on the display of the handheld device in response to meeting one or more return conditions.

2. The method of claim 1, further comprising:
launching in the handheld device a kiosk program; and
performing the process by the kiosk program.

3. The method of claim 2, wherein the process further comprises allowing launching one or more other programs while the allowable interfacing application is running and controlling the rights of said one or more other programs.

4. The method of claim 3, wherein the process further comprises allowing from the one or more other programs, when launched, access to the user interface except for a reserved portion of the user interface, which is kept reserved for the kiosk program.

5. The method of any one of preceding claims, wherein the process further comprises identifying the allowable interfacing application.

6. The method of any one of preceding claims, wherein the process further comprises receiving an identification of the allowable interfacing application.

7. The method of any one of preceding claims, wherein the providing of the user with the home screen comprising presenting one or more electronic case reporting questionnaires or links to one or more electronic case reporting questionnaires;

8. The method of any one of preceding claims , wherein the one or more return conditions comprise detecting that the user has used the return key.

9. The method of any one of preceding claims, wherein the one or more return conditions comprise that a timer has expired.

10. The method of any one of preceding claims, wherein the one or more return conditions comprise that a remote command has been received by the handheld device.

11. The method of any one of preceding claims, wherein the one or more return conditions comprise that the allowable interfacing application has ceased to use the display.

12. The method of any one of preceding claims, wherein the one or more return conditions comprises that a remote command has been received by the handheld device.

13. The method of any one of preceding claims, wherein the allowable interfacing application is configured to interface with external equipment.

14. A method for digital case reporting, comprising:
performing the method of claim 1; and
transferring data reported by the allowable interfacing application from a respective first back-end data storage to a second back-end data storage that differs from the first back-end storage.

15. A handheld device comprising at least one processor and a memory that comprises the computer executable program code which, when executed by the at least one processor, causes the handheld device to perform the method of any one of claims 1 to 14.
